# EUROPÄISCHE PATENTSCHRIFT

(11) **EP 0 481 527 B1**
(45) Veröffentlichungstag und Bekanntmachung des Hinweises auf die Patenterteilung: **03.05.1995**
(21) Anmeldenummer: 91118952.0
(22) Anmeldetag: 13.11.1990
(51) Int. Cl.: A61B 17/42, A61M 25/01

(54) **Instrumentensatz für den transvaginalen Gametentransfer und für die Tubenkathetisierung**
Set of instruments for the transvaginal gamete transfer and for catheterization
Jeu d'instruments pour le transfer de transvaginale de gamètes et pour cathétérisme

(30) Priorität: 04.12.1989 DE 3940064
(43) Veröffentlichungstag der Anmeldung: 22.04.1992
(62) Teilanmeldung aus: 90121663.0
(73) Patentinhaber: LABOTECT-LABOR-TECHNIK, GÖTTINGEN, GMBH, D-37120 Bovenden (DE)
(72) Erfinder: Schinkel, Otto, W-3406 Bovenden (DE); Norvath, Josef, W-3400 Göttingen (DE)
(74) Vertreter: Rehberg, Elmar, Dipl.-Ing.

(56) Entgegenhaltungen:
- DE-A- 2 727 273
- DE-A- 3 820 213
- US-A- 3 320 948
- US-A- 4 068 659
- US-A- 4 634 432

## Beschreibung

Die Erfindung bezieht sich auf einen Instrumentensatz für den transvaginalen Gametentransfer und für die Tubenkatheterisierung mit einem Katheterführungsrohr und einem Katheter, wobei das Katheterführungsrohr in seinem distalen Endbereich flexibel ausgebildet ist und dort eine Krümmung gegenüber seiner Achse aufweist.

Bei dem transvaginalen Gametentransfer als auch der Tubenkatheterisierung gilt es, einen Katheter durch die Scheide und den Uterus bis in die entsprechende Tube hinein einzuführen. Da die Katheter, die zum Einführen in die Tube geeignet sind, äußerst flexibel, nachgiebig und mit geringem Durchmesser ausgebildet sein müssen, bedient man sich zu ihrem Einführen bis in den Eingangsbereich der Tube eines Katheterführungsrohrs

Ein Instrumentensatz der eingangs beschriebenen Art ist aus der FR-A-26 35 453 bekannt, die erst nach dem effektiven Anmeldedatum veröffentlicht wurde und damit gemäß Artikel 54(2) EPÜ nicht zum Stand der Technik zählt. Hierbei ist das Katheterführungsrohr einstückig aus Kunststoff und an seinem distalen Ende spitz zulaufend ausgebildet. Zur Einführung des Katheterführungsrohrs bis in den Uterus hinein ist ein Mandrin vorgesehen, der das Katheterführungsrohr streckt. Hierbei verschwindet die Krümmung in dessen distalem Endbereich. Der distale Endbereich des Katheters ist im Durchmesser reduziert ausgebildet.

Bei einem weiteren Instrumentensatz der eingangs beschriebenen Art ist für das Katheterführungsrohr ein Mandrin vorgesehen, der dieselbe Krümmung wie das Katheterführungsrohr aufweist. Das Katheterführungsrohr ist wiederum einstückig aus Kunststoff und der Katheter in seinem distalen Endbereich im Durchmesser reduziert ausgebildet. Auf sein distales Ende zu verjüngt sich das Katheterführungsrohr. An seinem distalen Ende selbst weist es jedoch eine abgerundete Oberfläche auf. An dem Katheterführungsrohr ist eine verschiebbare Markierung als Orientierungsmarke vorgesehen. Der Katheter weist eine feste Markierung auf seiner Außenseite auf, die angibt, wann das distale Ende des Katheters aus dem Katheterführungsrohr heraustritt. Auch zum Einführen dieses Katheterführungsrohrs in den Uterus ist die Verwendung eines Mandrins vorgesehen. Es ist daher in diesem Stadium ebenfalls weitgehend unnachgiebig.

Aus der DE-A-27 27 273 ist ein chirurgischer Instrumentensatz für transurethrale Eingriffe bekannt. Dieser Instrumentensatz weist ein Führungsrohr und einen Obturator auf. Das Führungsrohr ist biegsam ausgebildet und weist in seinem distalen Endbereich eine Krümmung gegenüber seiner Achse auf. Der Obturator ist an seinem distalen Ende kugelförmig ausgebildet, wobei der Durchmesser der Kugel derart auf den Innendurchmesser des Führungsrohrs abgestimmt ist, daß der Obturator durch das Führungsrohr hindurchschiebbar ist. Der distale Endbereich des Obturators 6 kann von einem wendelförmig um einen biegbaren Kern gewickelten Band gebildet sein.

Aus der US-A-33 20 948 ist ein Instrumentensatz für transvaginale Eingriffe bekannt. Dieser Instrumentensatz umfaßt eine Hohlnadel und ein Führungsrohr. Das Führungsrohr weist ein distales Ende mit einem vergrößerten Durchmesser auf.

Aus der US-A-46 34 432 ist es bekannt, ein Katheterführungsrohr für die intravenöse Verwendung mit einer in die Wandung des Katheterführungsrohrs eingebetteten spiralförmig gewickelten Feder zu verstärken. Damit soll die Flexibilität des Katheterführungsrohrs nicht beeinträchtigt werden.

Der Erfindung liegt die Aufgabe zugrunde, einen Instrumentensatz der eingangs beschriebenen Art derart weiterzubilden, daß er zur verletzungsfreien Einlage des Katheterführungsrohrs geeignet ist.

Erfindungsgemäß wird dies dadurch erreicht, daß das Katheterführungsrohr an seinem distalen Ende kugelförmig ausgebildet ist und daß das Katheterführungsrohr in seinem distalen Endbereich aus einer spiralförmig gewickelten Bandkonstruktion besteht. Zumindest in seinem distalen Endbereich ist das Katheterführungsrohr spiralförmig gewickelt ausgebildet. So weist es dort eine enorme Flexibilität auf, und paßt sich den Krümmungen des Uterus besonders leicht an. Damit sind Furchungen und Verletzungen des Endometriums ausgeschlossen. Hierzu trägt auch das kugelförmige distale Ende des Katheterführungsrohrs bei. Der spiralförmig gewickelte Bereich kann etwa bis zur Hälfte der Gesamtlänge des Katheterführungsrohrs ausgedehnt sein.

Das Katheterführungsrohr kann in seinem proximalen Endbereich einen radial abgesetzten Flügel aufweisen, der entsprechend der Krümmung am Umfang des Katheterführungsrohrs angeordnet ist. Damit wird es für den Arzt möglich, die vordere Krümmung des Katheterführungsrohrs an seinem proximalen Ende zu verfolgen und ihre Lage abzulesen, so daß auch eine gezielte Drehung des distalen Endes des Katheterführungsrohrs möglich wird.

Das Katheterführungsrohr kann auf seiner Außenseite eine Skala als Orientierungshilfe aufweisen, die beispielsweise mit einer Klemmhalterung eines Spekulums zusammenarbeitet. Dem Arzt ist dadurch bei Mehrfachanwendung des Instrumentensatzes an ein der derselben Patientin die Möglichkeit gegeben, bei jeder weiteren Anwendung das Katheterführungsrohrs so einzuführen, daß sein vorderes Ende seinen ordnungsgemäßen Sitz erhält und nicht zu tief eingeführt wird.

Der Katheter kann auf seiner Außenseite eine mit dem proximalen Ende des Katheterführungsrohrs zusammenarbeitende Skala aufweisen. An dieser ist erkennbar, wieweit das vordere Ende des Katheters gegenüber dem vorderen Ende des Katheterführungsrohrs ausragt.

Der distale Endbereich des Katheters kann im Durchmesser reduziert ausgebildet sein. Auf diese Weise ist es möglich, durch den Katheter den isthmischen Tubenabschnitt mit geringem Widerstand zu erreichen. Hierbei ragt der Katheter aus dem Katheterführungsrohr heraus und wird ohne das Führungsrohr angewendet.

Die Erfindung wird anhand eines bevorzugten Ausführungsbeispiels weiter erläutert und beschrieben. Es zeigen:
- Figur 1: eine Darstellung des Instrumentensatzes bei der Anwendung und

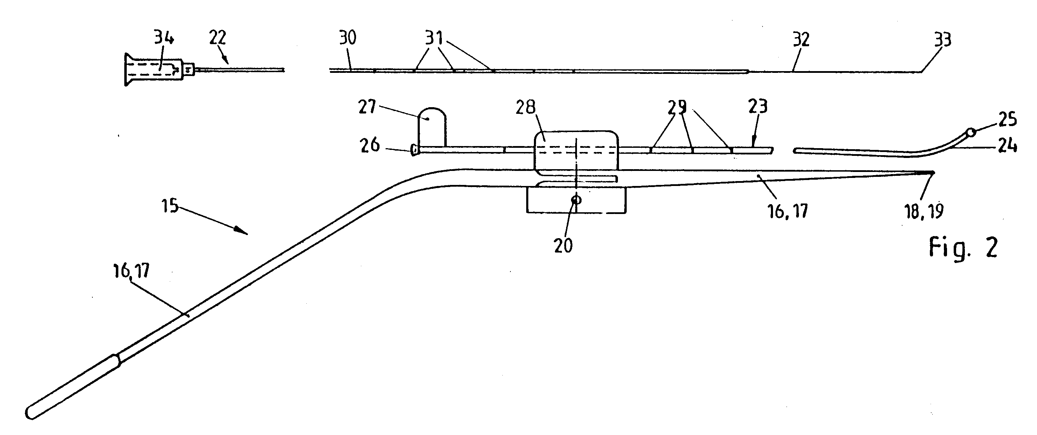

- Figur 2: die Teile des Instrumentensatzes.

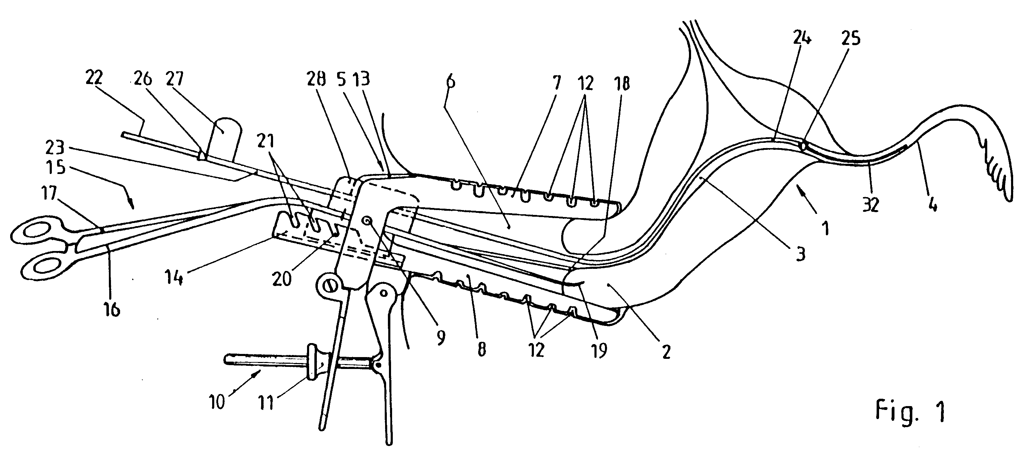

In Figur 1 ist schematisch und teilweise ein Uterus 1 mit Portio 2, Endometrium 3 und einer Tube 4 dargestellt. Ein Spekulum 5 ist in eine Scheide 6 eingeführt. Das Spekulum 5 weist einen Oberlöffel 7 und einen Unterlöffel 8 auf. Der Unterlöffel 8 ist über ein Gelenk 9 schwenkbar an dem Oberlöffel 7 gelagert. An dem Spekulum 5 ist eine Spreizeinrichtung 10 vorgesehen, mit deren Hilfe durch relatives Verdrehen einer Mutter 11 Oberlöffel 7 und Unterlöffel 8 gegeneinander gespreizt bzw. zusammengeführt werden können. Das Spekulum 5 ist in Figur 1 in der gewünschten gespreizten Lage dargestellt. Es wird in ungespreiztem Zustand in die Scheide 6 eingeführt, um 90° gedreht und dann mit der Spreizeinrichtung 15 gespreizt. Der Oberlöffel 7 und der Unterlöffel 8 weisen Aussparungen 12 auf, die randgeschlossen ausgebildet sind und die Form von langlochartigen Schlitzen haben. Beim Spreizen des Spekulums 5 legt sich die Wandung der Scheide 6 teilweise in die Aussparungen 16 und 17 hinein, wodurch das Spekulum seinen festen, unverrutschbaren Sitz erhält. Der Oberlöffel 7 weist an seinem hinteren Teil eine randoffene Ausnehmung 18 auf, wodurch der Einblicksbereich des Arztes auf die Portio 2 wesentlich verbessert wird.

An dem Spekulum 5 ist ein Widerlager 14 für eine Kugelzange 15 ausgebildet. Die Kugelzange 15 weist zwei Gelenkarme 16 und 17 auf, an deren vorderen Enden nach innen gerichtete Umbiegungen 18 und 19 vorgesehen sind. Mit den Umbiegungen 18 und 19 kann die Portio 2 ergriffen werden, um den Uterus 1 durch Ziehen an der Kugelzange 15 in eine weitgehend gestreckte Lage zu bringen. In dieser Stellung wird die Kugelzange 15 mit Hilfe eines Bolzens 20 in einen Schlitz 21 des Widerlagers 14 eingehängt. Zum eigentlichen transvaginalen Gametentransfer und zur Tubenkatheterisierung dient ein Katheter 22 mit Katheterführungsrohr 23. Das Katheterführungsrohr 23 weist in seinem distalen Endbereich eine vorgeformte Krümmung 24 auf. An seinem distalen Ende ist das Katheterführungsrohr 23 kugelförmig ausgebildet. Die Kugel 25 erleichtert das leichte Einführen des Katheterführungsrohr bis in den Eingangsbereich der Tube 4. Es versteht sich, daß das Katheterführungsrohr 23 hohl ausgebildet ist und das Hindurchschieben des Katheters 22 ermöglicht. Insbesondere in seinem distalen Endbereich, jedoch auch ausgedehnt bis zur Hälfte seiner Gesamtlänge, ist das Katheterführungsrohr 23 sehr biegsam ausgebildet und besteht aus einer gewickelten Bandkonstruktion, die diese weitgehende Flexibilität ermöglicht. An seinem proximalen Ende weist das Katheterführungsrohr einen Einführkonus 26 für den Katheter 22 auf. In dem proximalen Endbereich ist auch ein radial abgesetzter Flügel 27 vorgesehen, der so angeordnet ist, daß er mit der Krümmung 24 im distalen Endbereich des Katheterführungsrohrs 23 übereinstimmt.

An der Kugelzange 15 ist eine Klemmhalterung 28 vorgesehen, die in das Katheterführungsrohr 23 eingeklemmt werden kann. Auf diese Weise kann der Sitz des Katheterführungsrohrs 23 nach dem Einführen fixiert werden. Das Katheterführungsrohr 23 hat seinen ordnungsgemäßen Sitz dann erreicht, wenn sich die Kugel 25 am Eingang der Tube 4 befindet. Letztlich ist das Katheterführungsrohr 23 über die Kugelzange 15 an dem Spekulum 5 gelagert. Außen auf dem Katheterführungsrohr 23 ist eine Skala 29 angebracht, die mit der Klemmhalterung 28 zusammenarbeitet und der Orientierung dient.

Der Katheter 22 besteht im wesentlichen aus einem dünnen Kunststoffrohr 30 und ist sehr flexibel ausgebildet. Auf der Außenseite des Kunststoffrohrs 30 ist eine Skala 31, die mit dem proximalen Ende des Katheterführungsrohrs 23, also dem Einführkonus 26, zusammenarbeitet, vorgesehen. Das Kunststoffrohr 30 besitzt einen distalen Endteil 32, welcher gegenüber dem übrigen Teil des Kunststoffrohrs 30 einen reduzierten Durchmesser aufweist. Es handelt sich dabei um den Teil des Katheters 22, der über das distale Ende des Katheterführungsrohrs 23 hinausgeschoben wird, um durch den isthmischen Tubenabschnitt zu gelangen. Das distale Ende 33 des Katheters 22 ist abgerundet ausgebildet. Am proximalen Ende des Katheters 22 ist ein Anschluß 34 für eine Spritze vorgesehen.

Die Benutzung des Instrumentensatzes aus Katheter 22 und Katheterführungsrohr 23 geschieht wie folgt:
Zunächst wird das Katheterführungsrohr 23 über das Endometrium 3 bis zum Eingang der Tube 4 eingeführt. Das Katheterführungsrohr 23 kann beim Einführen feinfühlig geschoben werden. Durch Drehen des Katheterführungsrohrs 23 kann die Krümmung 24 so verlagert werden, daß das Einführen gegen einen möglichst geringen Widerstand und ohne jegliche Verletzungen geschieht. Der Flügel 27 zeigt dem Arzt an, nach welcher Seite die Krümmung 24 gerichtet ist. Hat das Katheterführungsrohr 23 seinen ordnungsgemäßen Sitz erreicht, so wird es in die Klemmhalterung 38 eingeklemmt, wobei sich der Arzt anhand der Skala 40 die ordnungsgemäße Lage und damit die Einführtiefe des Katheterführungsrohr 23 merken kann. Dies ist für einen Zweit- oder Mehrfacheinsatz des Instrumentensatzes bei der gleichen Patientin sinnvoll. Nachdem der Sitz des Katheterführungsrohrs 23 fixiert ist, wird der Katheter 22 mit seinem distalen Ende 44 in den Einführkonus 26 des Katheterführungsrohrs 23 eingeführt und zunächst soweit durchgeschoben, bis sich das distale Ende 33 des Katheters 22 an der Kugel 25 befindet. Dies wird dem Arzt durch den Beginn der Skala 31 angezeigt, deren erster Strich sich dann an dem Einführkonus 26 befindet. Jeder weitere Strich der Skala 31 zeigt dem Arzt dann an, daß das distale Ende 33 bzw. der Endteil 32 beispielsweise um einen weiteren Zentimeter aus dem Katheterführungsrohr 23 ausgetreten ist und die Kugel 25 überragt. Dabei wird auch hier der Katheter 22 feinfühlig verschoben, bis der Endteil 32 den isthmischen Tubenabschnitt durchsetzt. Nun kann sich der Arzt anhand der Skala 31 diese Lage, die spezifisch für die betreffende Patientin ist, merken oder notieren. Anschließend kann der transvaginale Gametentransfer erfolgen.

### Bezugszeichenliste:

- 1: = Uterus
- 2: = Portio
- 3: = Endometrium
- 4: = Tube
- 5: = Spekulum
- 6: = Scheide
- 7: = Oberlöffel
- 8: = Unterlöffel
- 9: = Gelenk
- 10: = Spreizeinrichtung
- 11: = Mutter
- 12: = Aussparung
- 13: = Ausnehmung
- 14: = Widerlager
- 15: = Kugelzange
- 16: = Gelenkarm
- 17: = Gelenkarm
- 18: = Umbiegung
- 19: = Umbiegung
- 20: = Bolzen
- 21: = Schlitz
- 22: = Katheter
- 23: = Katheterführungsrohr
- 24: = Krümmung
- 25: = Kugel
- 26: = Einführkonus
- 27: = Flügel
- 28: = Klemmhalterung
- 29: = Skala
- 30: = Kunststoffrohr
- 31: = Skala
- 32: = Endteil
- 33: = Ende
- 34: = Anschluß

## Patentansprüche

1. Instrumentensatz für den transvaginalen Gametentransfer und für die Tubenkatheterisierung mit einem Katheterführungsrohr und einem Katheter, wobei das Katheterführungsrohr in seinem distalen Endbereich eine Krümmung gegenüber seiner Achse aufweist und an seinem distalen Ende verdickt ist,
dadurch gekennzeichnet, dass
die Verdickung des distalen Endes kugelfömig ausgebildet ist, und das Katheterführungsrohr in seinem distalen Endbereich flexibel ist und aus einer spiralförmig gewickelten Bandkonstruktion besteht.

2. Instrumentensatz nach Anspruch 1, dadurch gekennzeichnet, daß das Katheterführungsrohr (23) in seinem proximalen Endbereich einen radial abgesetzten Flügel (27) aufweist, der entsprechend der Krümmung (26) am Umfang des Katheterführungsrohrs (23) angeordnet ist.

3. Instrumentensatz nach einem der voranstehenden Ansprüche 1 und 2, dadurch gekennzeichnet, daß das Katheterführungsrohr (23) auf seiner Außenseite eine Skala (29) als Orientierungshilfe aufweist.

4. Instrumentensatz nach einem der voranstehenden Ansprüche 1 bis 3, dadurch gekennzeichnet, daß der Katheter (22) auf seiner Außenseite eine mit dem proximalen Ende des Katheterführungsrohrs (23) zusammenarbeitende Skala (31) aufweist.

5. Instrumentensatz nach einem der voranstehenden Ansprüche 1 bis 4, dadurch gekennzeichnet, daß der distale Endbereich des Katheters im Durchmesser reduziert ausgebildet ist.

## Claims

1. Set of instruments for the transvaginal gamete transfer and for tube catheteration comprising a catheter guide tube and a catheter, wherein the catheter guide tube comprises in its distal end region a curvature with regard to its axis and is bulged at its distal end, **characterized in that** the bulge of the distal end is formed ball-like and that the catheter guide tube is flexible in its distal end region and is comprised by a spirally rolled ribbon construction.

2. Set of instruments according to claim 1, **characterized in that** the catheter guide tube (23) in its proximal end region comprises a radially offset wing (27) which is corresponding to the curvature (26) disposed on the circumference of the catheter guide tube (23).

3. Set of instruments according to one of the preceding claims 1 and 2, **characterized in that** the catheter guide tube (23) comprises on its outside a scale (29) for orientation purposes.

4. Set of instruments according to one of the preceding claims 1 to 3, **characterized in that** the catheter (22) comprises on its outside a scale (31) cooperating with the proximal end of the catheter guide tube (23).

5. Set of instruments according to one of the preceding claims 1 to 4, **characterized in that** the distal end portion of the catheter is reduced in diameter.

## Revendications

1. Ensemble d'instruments pour le transfert transvaginal de gamètes et pour le cathétérisme des trompes comportant un tube de guidage de cathéter et un cathéter, le tube de guidage de cathéter présentant dans sa zone d'extrémité distale une courbure par rapport à son axe et étant surépaissi à son extrémité distale, caractérisé en ce que la surépaisseur de l'extrémité distale est sphérique, et le tube de guidage de cathéter est flexible dans sa zone d'extémité distale et est une construction en bande enroulée en spirale.

2. Ensemble d'instruments selon la revendication 1, caractérisé en ce que le tube de guidage de cathéter (23) présente dans sa zone d' extrémité proximale, une ailette (27) avec un décrochement radial, qui est disposée suivant la courbure (26), sur le pourtour du tube de guidage de cathéter (23).

3. Ensemble d'instruments selon l'une des revendications 1 et 2 précédentes, caractérisé en ce que le tube de guidage de cathéter (23) présente sur son côté extérieur une é'chelle graduée (29) favorisant l'orientation.

4. Ensemble d'instruments selon l'une des revendications 1 à 3 précédentes, caractérisé en ce que le cathéter (22) présente sur son côté extérieur une échelle graduée (31) coopérant avec l'extrémité proximale du tube de guidage de cathéter (23).

5. Ensemble d'instruments selon l'une des revendications 1 à 4 précédentes, caractérisé en ce que la zone d'extrémité distale du cathéter présente un diamètre réduit.
